(19) Europäisches Patentamt
European Patent Office
Office européen des brevets

(11) EP 3 889 125 A1

(12) **EUROPEAN PATENT APPLICATION**
published in accordance with Art. 153(4) EPC

(43) Date of publication:
**06.10.2021 Bulletin 2021/40**

(21) Application number: **19889569.0**

(22) Date of filing: **26.11.2019**

(51) Int Cl.:
***C07C 41/08*** *(2006.01)* ***C07C 43/16*** *(2006.01)*
***C07B 61/00*** *(2006.01)* ***B01J 23/04*** *(2006.01)*

(86) International application number:
**PCT/JP2019/046071**

(87) International publication number:
**WO 2020/111030 (04.06.2020 Gazette 2020/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2018 JP 2018221035**

(71) Applicant: **Maruzen Petrochemical Co., Ltd.**
**Tokyo 104-8502 (JP)**

(72) Inventors:
- **NANIKI, Takashi**
  **Ichihara-shi, Chiba 290-8503 (JP)**
- **ITO, Jun**
  **Ichihara-shi, Chiba 290-8503 (JP)**
- **HASHIMA, Yuji**
  **Ichihara-shi, Chiba 290-8503 (JP)**
- **TSUCHIDA, Wataru**
  **Ichihara-shi, Chiba 290-8503 (JP)**
- **TAKAHASHI, Takashi**
  **Ichihara-shi, Chiba 290-8503 (JP)**
- **TAKAYAMA, Ayato**
  **Ichihara-shi, Chiba 290-8503 (JP)**
- **SATO, Tomohiko**
  **Ichihara-shi, Chiba 290-8503 (JP)**

(74) Representative: **Hartz, Nikolai**
**Wächtershäuser & Hartz**
**Patentanwaltspartnerschaft mbB**
**Weinstrasse 8**
**80333 München (DE)**

# (54) METHOD FOR PRODUCING DIVINYL ETHER COMPOUND HAVING ALKYLENE SKELETON

(57) Provided is a method capable of producing a divinyl ether compound having an alkylene skeleton from an alkanediol and acetylene at a rapid production rate and a high reaction yield. In the production method, a compound represented by formula (1) is reacted with acetylene in the presence of an alkali metal catalyst to produce a compound represented by formula (2), and the reaction is performed in the absence of a solvent.

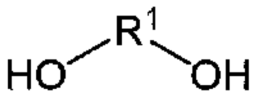
(1)

In formula (1), $R^1$ represents an alkylene group having 4 to 20 carbon atoms.

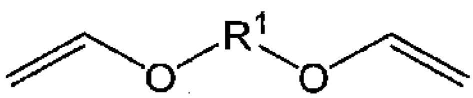
(2)

In formula (2), $R^1$ has the same meaning as $R^1$ in formula (1).

EP 3 889 125 A1

## Description

### Technical Field

The present invention relates to a method for producing a divinyl ether compound having an alkylene skeleton.

### Background Art

A compound having two vinyl ether structures is called as a divinyl ether compound, and is used as a crosslinking component and a curing component of a raw material for a polymerization composition. Of this, the divinyl ether compound having an alkylene skeleton is expected to be applied to, for example, adhesives, paints, printing inks, and resist materials because of its excellent properties such as low toxicity, low irritation, and low shrinkage.

Leppe method is known as a method for producing a divinyl ether compound, in which an alcohol (diol) is reacted with acetylene in the presence of an alkali metal catalyst. For example, Patent Literature 1 discloses a method for separating and collecting diethylene glycol divinyl ether by reacting diethylene glycol and acetylene in the presence of diethylene glycol divinyl ether, proceeding with the reaction to the state where both diethylene glycol monovinyl ether and diethylene glycol divinyl ether are produced, and then performing extractive distillation.

In the production method described in Patent Literature 1, diethylene glycol divinyl ether can be collected with high purity. However, the reaction to diethylene glycol divinyl ether does not completely proceed, and diethylene glycol monovinyl ether is produced in a high proportion (about 30% by mass). Therefore, it considers that the reaction yield of diethylene glycol divinyl ether per reaction is low and the production efficiency will be impaired in practical use. In addition, this method has not realized the production of a divinyl ether compound having an alkylene skeleton.

Further, Patent Literature 2 discloses a method for producing a divinyl ether compound having an alkylene skeleton from an alkanediol and acetylene in dimethyl sulfoxide as an aprotic polar solvent by the Leppe method. However, this production method has a problem of low production speed (production rate) of a divinyl ether compound.

Meanwhile, as a method which differs from the Leppe method and which manufactures a divinyl ether compound having an alkylene skeleton, it has reported a method for performing a vinyl group exchange reaction by using a diol and vinyl acetate (Patent Literature 3). However, this method is problematic that the production speed of the divinyl ether compound is low, and the production cost increases because an expensive iridium catalyst is used for the reaction.

### Citation List

#### Patent Literature

Patent Literature 1: JP 2014-513048 A
Patent Literature 2: WO 2015/190376 A
Patent Literature 3 JP 2017-68246 A

### Summary of Invention

#### Technical Problem

An object of the present invention is to provide a method capable of producing a divinyl ether compound having an alkylene skeleton from an alkanediol and acetylene at a rapid production rate and a high reaction yield.

#### Solution to Problem

As a result of intensive studies to solve the above problems, the present inventors found that a divinyl ether compound having an alkylene skeleton can be produced at a rapid production rate and a high reaction yield by the reaction of an alkanediol with acetylene in the absence of a solvent, and consequently completed the present invention.

That is, the present invention provides the following <1> to <6>.

<1> A method for reacting a compound represented by formula (1) (hereinafter, also referred to as alkanediol (1)) with acetylene by using an alkali metal catalyst to produce a compound represented by formula (2) (hereinafter, also referred to as divinyl ether compound (2)), in which the reaction is performed in the absence of a solvent (hereinafter, also referred to as the production method of the present invention).

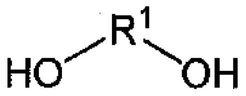

(1)

In formula (1), $R^1$ represents an alkylene group having 4 to 20 carbon atoms.

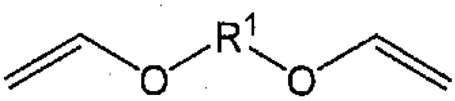

(2)

In formula (2), $R^1$ has the same meaning as $R^1$ in formula (1).

<2> The production method according to <1>, wherein the compound represented by formula (1) is represented by formula (3), (4), or (5).

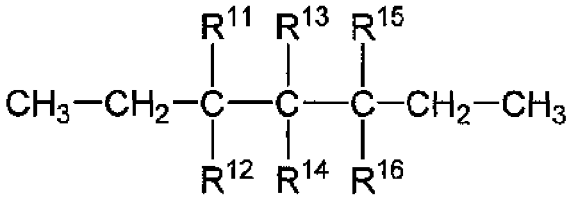

(3)

In formula (3), any two of $R^{11}$ to $R^{16}$ represent a hydroxy group or a hydroxymethyl group, and the others represent a hydrogen atom.

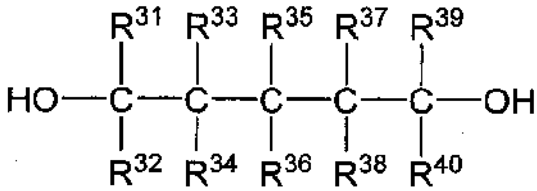

(4)

In formula (4), any one of $R^{31}$ to $R^{40}$ represents a methyl group, and the others represent a hydrogen atom.

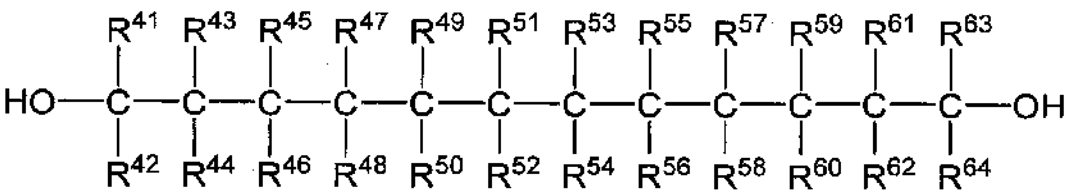

(5)

In formula (5), $R^{41}$ to $R^{64}$ represent a hydrogen atom or an alkyl group having 1 to 8 carbon atoms, provided that as for the combination of $R^{41}$ to $R^{64}$, any one of $R^{41}$ to $R^{64}$ is an alkyl group having 1 to 8 carbon atoms and the others are hydrogen atoms, or all of $R^{41}$ to $R^{64}$ are hydrogen atoms.

<3> The production method according to <1> or <2>, wherein the compound represented by formula (1) is at least one selected from the group consisting of 3-methyl-1,5-pentanediol, 2,4-diethyl-1,5-pentanediol, 2-butyl-2-ethyl-1,3-propanediol, 1,12-dodecanediol, and 1,12-octadecanediol.

<4> The production method according to any one of <1> to <3>, wherein the reaction is performed in a range of 50 to 170°C.

<5> The production method according to any one of <1> to <4>, wherein the alkali metal catalyst is at least one selected from the group consisting of an alkali metal hydroxide and an alkali metal carbonate.

<6> The production method according to any one of <1> to <5>, wherein an amount of the alkali metal catalyst used

is in a range of 1 to 60 mol with respect to 100 mol of the compound represented by the formula (1).

Advantageous Effects of Invention

According to the present invention, it is possible to produce a divinyl ether compound having an alkylene skeleton from an alkanediol and acetylene at a rapid production rate and a high reaction yield.

Description of Embodiments

In the production method of the present invention, an alkanediol (1) is reacted with acetylene by using an alkali metal catalyst in the absence of a solvent to produce a divinyl ether compound (2).

In formulas (1) and (2), the alkylene group (alkanediyl group) represented by $R^1$ has carbon atoms of 4 to 20, preferably 6 to 18 from the viewpoint of enhancing the desired effect of the present invention. In addition, the alkylene group may be linear or branched.

Specific examples of the alkylene group include butane-1,1-diyl group, butane-1,2-diyl group, butane-1,3-diyl group, butane-1,4-diyl group, pentane-1,1-diyl group, pentane-1,2-diyl group, pentane-1,3-diyl group, pentane-1,4-diyl group, pentane-1,5-diyl group, hexane-1,1-diyl group, hexane-1,2-diyl group, hexane-1,3-diyl group, hexane-1,4-diyl group, hexane-1,5-diyl group, hexane-1,6-diyl group, 3-methyl-pentane-1,5-diyl group, heptane-1,7-diyl group, octane-1,8-diyl group, nonane-1,9-diyl group, 2,4-diethyl-pentane-1, 5-diyl group, 2-butyl-2-ethyl-propane-1,3-diyl group, decane-1,10-diyl group, undecane-1,11-diyl group, dodecane-1,12-diyl group, tridecane-1,13-diyl group, tetradecane-1,14-diyl group, pentadecane-1,15-diyl group, hexadecane-1,16-diyl group, heptadecane-1,17-diyl group, octadecane-1,12-diyl group, and octadecane-1,18-diyl group.

Alkanediol (1) used in the present invention preferably has a boiling point of 50°C or more at normal pressure, and more preferably a boiling point of 100 to 450°C at normal pressure.

In addition, alkanediol (1) is preferably represented by the following formulas (3) to (5) from the viewpoints of enhancing the desired effect of the present invention and the usefulness of the corresponding divinyl ether compound as a material. In addition, examples of the alkanediol represented by formula (3) include those represented by formulas (3-1) to (3-3). The production method of the present invention can provide the corresponding divinyl ether compound from the alkanediol having such a structure.

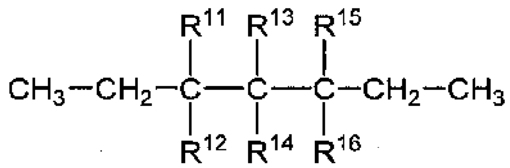

(3)

In formula (3), any two of $R^{11}$ to $R^{16}$ represent a hydroxy group or a hydroxymethyl group, and the others represent a hydrogen atom.

When representing a hydroxy group or a hydroxymethyl group, $R^{11}$ to $R^{16}$ may be the same or different from each other.

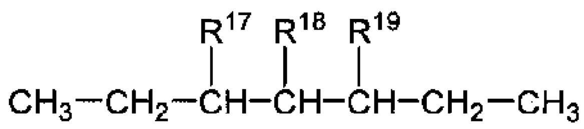

(3-1)

In formula (3-1), any two of $R^{17}$ to $R^{19}$ represent a hydroxy group or a hydroxymethyl group, and the others represent a hydrogen atom.

When representing a hydroxy group or a hydroxymethyl group, $R^{17}$ to $R^{19}$ may be the same or different from each other.

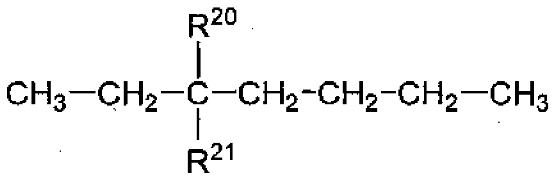

(3-2)

In formula (3-2), $R^{20}$ and $R^{21}$ each independently represent a hydroxy group or a hydroxymethyl group.

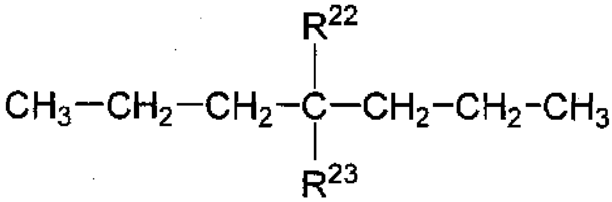

(3-3)

In formula (3-3), $R^{22}$ and $R^{23}$ each independently represent a hydroxy group or a hydroxymethyl group.

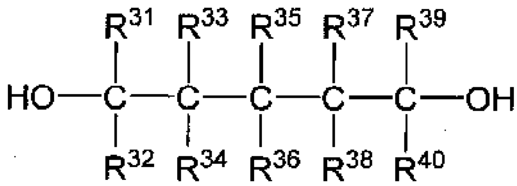

(4)

In formula (4), any one of $R^{31}$ to $R^{40}$ represents a methyl group, and the others represent a hydrogen atom.

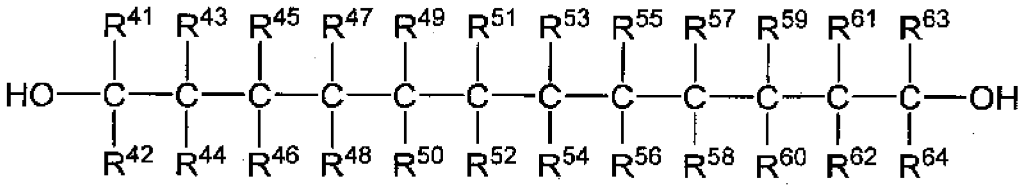

(5)

In formula (5), $R^{41}$ to $R^{64}$ represent a hydrogen atom or an alkyl group having 1 to 8 carbon atoms, provided that as for the combination of $R^{41}$ to $R^{64}$, any one of $R^{41}$ to $R^{64}$ is an alkyl group having 1 to 8 carbon atoms and the others are hydrogen atoms, or all of $R^{41}$ to $R^{64}$ are hydrogen atoms.

The alkyl groups represented by $R^{41}$ to $R^{64}$ may be linear or branched, and examples thereof include a methyl group, an ethyl group, a n-propyl group, an isopropyl group, a n-butyl group, an isobutyl group, a sec-butyl group, a tert-butyl group, a n-pentyl group, an isopentyl group, a neopentyl group, a n-hexyl group, an isohexyl group, a n-heptyl group, and a n-octyl group.

Specific examples of the alkanediol (1) include 1,2-butanediol, 1,3-butanediol, 1,4-butanediol, 2,3-butanediol, 2-methyl-1,3-propanediol, 1,2-pentanediol, 1,4-pentanediol, 1,5-pentanediol, 2,4-pentanediol, 2-methyl-1,3-butanediol, 3-methyl-1,3-butanediol, 2-methyl-2,3-butanediol, 2,2-dimethyl-1,3-propanediol, 1,2-hexanediol, 1,5-hexanediol, 1,6-hexanediol, 2,4-hexanediol, 2,5-hexanediol, 3-methyl-1,5-pentanediol, 4-methyl-2,3-pentanediol, hexylene glycol, 3,3-dimethyl-1,2-butanediol, 2,2-dimethyl-1,3-butanediol, pinacol, 2-ethyl-2-methyl-1,3-propanediol, 1,2-heptanediol, 1,4-heptanediol, 1,7-heptanediol, 3,3-heptanediol, 3,4-heptanediol, 3,5-heptanediol, 4,4-heptanediol, 5-methyl-2,4-hexanediol, 3,3-dimethyl-1,5-pentanediol, 2,4-dimethyl-2,4-pentanediol, 2,2-diethyl-1,3-propanediol, 2-methyl-2-propyl-1,3-propanediol, 1,2-octanediol, 1,8-octanediol, 4,5-octanediol, 3-methyl-2,4-heptanediol, 2-ethyl-1,2-hexanediol, 2-ethyl-1,3-hexanediol, 2-ethyl-1,4-hexanediol, 2,5-dimethyl-2,5-hexanediol, 2-propyl-1,2-pentanediol, 2,4,4-trimethyl-1,2-pentanediol, 2-propyl-1,3-pentanediol, 2-methyl-2-(1-methylpropyl)-1,3-propanediol, 2,2,4-trimethyl-1,3-pentanediol, 2,4,4-trimethyl-2,3-pentanediol, 1,2-nonanediol, 1,9-nonanediol, 2,4-diethyl-1,5-pentanediol, 2-ethyl-3-propyl-1,4-butanediol, 2-butyl-2-ethyl-1,3-propanediol, 1,2-decanediol, 1,10-decanediol, 2,7-dimethyl-2,7-octanediol, 3,6-dimethyl-3,6-octanediol, 2,3,4,5-tetramethyl-3,4-hexanediol,2,2-dibutyl-1,3-propanediol, 2,2-diisobutyl-1,3-propanediol, 1,2-dodecanediol, 1,12-dodecanediol, 5-ethyl-3-methyl-2,4-nonanediol, 7-ethyl-2-methyl-4,6-nonanediol, 2-butyl-1,3-octanediol, 2-methyl-2,3-dodecanediol, 2,2-diisoamyl-1,3-propanediol, 2-(4,4-dimethylpentyl)-2-propyl-1,3-propanediol, 1,2-tetradecanediol, 1,14-tetradecanediol, 2,2,9,9-tetramethyl-1,10-decandiol, 2-octyl-2-propyl-1,3-propanediol, 1,2-hexadecanediol, 1,16-hexadecanediol, 2-decyl-2-propyl-1,3-propanediol, 2-(2-methylpropyl)-2-nonyl-1,3-propanediol, 5,13-heptadecanediol, 2-dodecyl-2-ethyl-1,3-propanediol, 1,12-octadecanediol, 2,9-dimethyl-2,9-dipropyl-1,10-decanediol, 2-dodecyl-2-propyl-1,3-propanediol, 2,2-dioctyl-1,3-propanediol, and 8-ethyl-1,18-octadecanediol.

Of these, preferable is at least one selected from the group consisting of 3-methyl-1,5-pentanediol, 2,4-diethyl-1,5-pentanediol, 2-butyl-2-ethyl-1,3-propanediol, 1,12-dodecanediol, and 1,12-octadecanediol.

The amount of alkanediol (1) used is preferably 85 to 99% by mass, and more preferably 90 to 99% by mass with respect to the total amount of the liquid phase (excluding the product) in the reaction system from the viewpoints of production cost and purification of the divinyl ether compound to be produced.

In addition, the total charging ratio of alkanediol (1) and the alkali metal catalyst is preferably 95 to 100% by mass, and more preferably 97.5 to 100% by mass, with respect to the total of the charged components (excluding acetylene).

The alkali metal catalyst used in the present invention is not particularly limited, and conventionally known catalysts can be used. Examples thereof include alkali metal hydroxides such as potassium hydroxide and sodium hydroxide; and alkali metal carbonates such as potassium carbonate and sodium carbonate. The alkali metal catalyst may be used singly or in combination of two or more. Of these, alkali metal hydroxides are preferable from the viewpoint of enhancing the desired effects of the present invention.

The amount of the alkali metal catalyst used is preferably in the range of 1 to 60 mol, and more preferably in the range of 5 to 45 mol with respect to 100 mol of alkanediol (1), from the viewpoints of enhancing the desired effect of the present invention and production cost.

The supply pressure of acetylene in the production method of the present invention is not particularly limited, and from the viewpoints of safety and reaction progress, it is preferably 0.01 to 0.4 MPa, and more preferably 0.01 to 0.08 MPa, in terms of the gauge pressure.

The reaction temperature of the reaction between alkanediol (1) and acetylene is preferably in the range of 50 to 170°C, and more preferably in the range of 90 to 160°C, from the viewpoints of safety and reaction progress. When alkanediol (1) is pretreated with an alkali metal catalyst to be alkoxided prior to the supply of acetylene, the alkoxide reaction can also be performed at the same reaction temperature.

The reaction time of the reaction between alkanediol (1) and acetylene may be adjusted according to the type of alkanediol (1) for example, and is typically 1 to 72 hours, and preferably 1.5 to 48 hours.

In addition, the reaction of alkanediol (1) with acetylene can be performed by a batch method, a semi-continuous method, or a continuous method.

In addition, the order of contact of alkanediol (1), the acetylene, and the alkali metal catalyst is arbitrary. For example, it includes a method in which alkanediol (1), acetylene, and an alkali metal catalyst are supplied into the reactor and then the divinyl etherification reaction is conducted, or a method in which alkanediol (1) and an alkali metal catalyst are previously charged in the reactor, the temperature is raised to a predetermined temperature, and then the acetylene pressure in the reactor is increased to initiate the reaction. When alkanediol (1) and the alkali metal catalyst are previously charged in the reactor, the alkoxide reaction may proceed prior to the supply of acetylene. The reaction pressure of the alkoxide reaction is preferably 1 to 101.3 kPa in terms of absolute pressure.

After completion of the reaction between alkanediol (1) and acetylene, the obtained divinyl ether compound (2) can be treated and isolated by a known operation and treatment method. For example, the catalyst is separated by filtration and then targeted divinyl ether compound (2) can be isolated by distillation. The divinyl ether compound (2) is derived from alkanediol (1) and has a corresponding chemical structure.

According to the present invention, it is possible to produce the divinyl ether compound (2) from alkanediol (1) and acetylene at a rapid production rate and a high reaction yield. In addition, due to a solvent-free reaction, it can easily provide divinyl ether compound (2) at low cost without adding not only aprotic polar solvents such as dimethyl sulfoxide, but also compounds that are liquid at room temperature other than alkanediol (1), such as divinyl ether compounds corresponding to alkanediol (1) used as a raw material (divinyl ether compound having the same chemical structure as targeted divinyl ether compound (2)).

## Example

Hereinafter, the present invention will be described in detail with reference to examples; however, the present invention is not limited to these examples. The measurement in the following examples was performed in accordance with the following measurement method.

Composition analysis of reaction solution

The composition of the reaction solution was analyzed by gas chromatography. The analysis conditions were as follows.

Equipment: Product name "GC-2010 Plus" (manufactured by Shimadzu Corporation)
Detector: FID
Column: DB-5 (30m, 0.25mmID, 1.0μm, manufactured by Agilent Technologies, Inc.)

• 1,12-Octadecane diol divinyl ether (ODDVE)

INJ temperature: 250°C
DET temperature: 250°C
Split ratio: 50
Column temperature: Holding for 3 min at 180°C → Heating up to 250°C at 10°C/min → Holding for 20 min at 250°C (30 min in total)

• 2,4-Diethyl-1,5-pentanediol divinyl ether (DEPDVE)

INJ temperature: 250°C
DET temperature: 300°C
Split ratio: 50
Column temperature: Heating up from 100°C to 160°C at 4°C/min → Holding for 10 min at 160°C → Heating up to 300°C at 20°C/min → Holding for 13 min at 300°C (45 min in total)

• 1,12-Dodecane diol divinyl ether (3DVE)

INJ temperature: 250°C
DET temperature: 270°C
Split ratio: 50
Column temperature: Holding for 3 min at 180°C → Heating up to 250°C at 5°C/min → Holding for 13 min at 250°C (30 min in total)

• 3-Methyl-1,5-pentanediol divinyl ether (MPDVE)

INJ temperature: 250°C
DET temperature: 300°C
Split ratio: 50
Column temperature: Holding 5 min at 100°C → Heating up to 280°C at 10°C/min → Holding 7 min at 280°C (30 min in total)

Potassium concentration measurement

The potassium concentration of the reaction solution was measured by the following equipment.

Equipment: Product name "Automatic titrator COM-1700A" (manufactured by HIRANUMA SANGYO Co., Ltd.)
Titrant: 0.2 mol/L hydrochloric acid and ethanol

Measurement of moisture content of distillate

The moisture content of the distillate was measured by using the following equipment.

Equipment: Product name "automatic moisture measuring equipment AQV-300" (manufactured by HIRANUMA SANGYO Co., Ltd.)
Titrant: HYDRANAL Composite 5K (manufactured by HAYASHI PURE CHEMICAL IND., LTD.)
Titration solvent: Karl Fischer reagent HAYASHI-solvent CE (manufactured by HAYASHI PURE CHEMICAL IND., LTD.)

Measurement of reaction rate

The reaction rate was calculated by the following formula.

$$r1 = M/\{(t1)\times V\}$$

r1: Reaction rate (g/L·hr)
M: Divinyl ether production amount (g)
t1: Reaction time (hr)
V: Reaction volume (L)

Measurement of production rate in reaction step

The production rate in the reaction step was calculated by the following formula.

$$r2 = M/\{(t1+t2)\times V\}$$

r2: Production rate in reaction step (g/L·hr)
M: Divinyl ether production amount (g)
t1: Reaction time (hr)
t2: Time of preparing potassium alkoxide (hr)
V: Reaction volume (L)

Example 1: Synthesis of 1,12-octadecanediol divinyl ether (ODDVE)

0.18 kg (3.3 mol) of potassium hydroxide (manufactured by Nippon Soda Co., Ltd.) and 3.50 kg (12.2 mol) of 1,12-octadecanediol (manufactured by KOKURA SYNTHETIC INDUSTRIES, LTD.) that had been previously dissolved by heating were charged in a pressure-resistant reaction vessel made of SUS316, having a capacity of 10L, and equipped with a stirrer, pressure gauge, thermometer, gas introduction pipe, gas purge line, decompression line, and liquid sampling line, and nitrogen was purged inside the vessel.

After nitrogen purge, the temperature inside the vessel was raised to 150°C and stirring was performed at 250 rpm. The pressure inside the vessel was gradually reduced to 3 kPaA (A indicates absolute pressure), and bubbling with 0.1 NL/min nitrogen was performed for 3 hr from the liquid sampling line. Through the above operation, 0.06 kg of a distillate including water as the main component was taken out. Thus, potassium alkoxide derived from 1,12-octadecanediol was obtained.

Then, the inside of the vessel was purged with acetylene while stirring at 420 rpm. Acetylene was continuously supplied, the inside of the vessel was kept at 0.03 MPaG (G indicates gauge pressure) and 150°C, and the reaction was performed for 11.5 hr. As a result of gas chromatography analysis of the reaction solution, the conversion rate was 99% or more, and the selectivity was 97%. The reaction rate in this case was calculated to be 61 g/L·hr, and the production rate in reaction step was calculated to be 49 g/L·hr.

This reaction solution was subjected to filtration under pressure at 120°C and a nitrogen pressure of 0.1 MPaG by using a filter cloth (air permeability: 0.6 to 1.8 cc/cm$^2$·sec, made of polyphenylene sulfide resin) to separate a catalyst. The potassium concentration in the filtrate was 0.4% by mass. Moreover, simple distillation of the filtrate was performed under a reduced pressure of 0.02 kPaA to obtain 3.37 kg (10.0 mol) of 1,12-octadecanediol divinyl ether (hereinafter referred to as ODDVE). The obtained ODDVE had a purity of 99% or more and a yield of 81%. The results are shown in Table 1.

Example 2: Synthesis of 2,4-diethyl-1,5-pentanediol divinyl ether (DEPDVE)

Into the same reaction vessel as in Example 1, 3.80 kg (23.7 mol) of 2,4-diethyl-1,5-pentanediol (manufactured by KH Neochem Co., Ltd.) and 0.29 kg (5.2 mol) of potassium hydroxide were charged, and nitrogen was purged inside the vessel.

After nitrogen purge, the temperature inside the vessel was raised to 150°C and stirring was performed at 250 rpm. Bubbling with 1 NL/min nitrogen was performed for 3 hr from the liquid sampling line. Through the above operation, 0.09 kg of a distillate including water as the main component was taken out. Thus, potassium alkoxide derived from 2,4-diethyl-1,5-pentanediol was obtained.

Then, the temperature in the vessel was raised to 155°C, and the inside of the vessel was purged with acetylene while stirring at 420 rpm. Acetylene was continuously supplied, the inside of the vessel was kept at 0.03 MPaG and 155°C, and the reaction was performed for 11.5 hr. As a result of gas chromatography analysis of the reaction solution, the conversion rate was 99% or more, and the selectivity was 97%. The reaction rate in this case was calculated to be 60 g/L·hr, and the production rate in reaction step was calculated to be 47 g/L·hr.

This reaction solution was subjected to filtration under pressure at 120°C and a nitrogen pressure of 0.1 MPaG by using the same filter cloth as in Example 1 to separate a catalyst. The potassium concentration in the filtrate was 0.2% by mass. Moreover, fine distillation of the filtrate was performed by using a distillation column manufactured by Kiriyama Glass Works Co. with 5 theoretical stages under the conditions of a pressure of 1.3 kPaA and a reflux ratio of 1 to obtain 3.50 kg (16.5 mol) of 2,4-diethyl-1,5-pentanediol divinyl ether (hereinafter referred to as DEPDVE). The obtained DEPDVE had a purity of 99% or more and a yield of 70%. The results are shown in Table 1.

Example 3: Synthesis of 1,12-dodecanediol divinyl ether (3DVE)

Into the same reaction vessel as in Example 1, 0.23 kg (4.1 mol) of potassium hydroxide and 3.96 kg (19.6 mol) of 1,12-dodecanediol (manufactured by FUJIFILM Wako Chemical Corporation) that had previously dissolved by heating were charged, and nitrogen was purged inside the vessel.

After nitrogen purge, the temperature inside the vessel was raised to 135°C and stirring was performed at 250 rpm. The pressure inside the vessel was gradually reduced to 20 kPaA, and bubbling with 0.1 NL/min nitrogen was performed for 3 hr from the liquid sampling line. Through the above operation, 0.09 kg of a distillate including water as the main component was taken out. Thus, potassium alkoxide derived from 1,12-dodecanediol was obtained.

Then, the temperature in the vessel was raised to 155°C, and the inside of the vessel was purged with acetylene while stirring at 420 rpm. Acetylene was continuously supplied, the inside of the vessel was kept at 0.03 MPaG and 155°C, and the reaction was performed for 17 hr. As a result of gas chromatography analysis of the reaction solution, the conversion rate was 99% or more, and the selectivity was 94%. The reaction rate in this case was calculated to be 52 g/L·hr, and the production rate in reaction step was calculated to be 44 g/L·hr.

This reaction solution was subjected to filtration under pressure at 120°C and a nitrogen pressure of 0.1 MPaG by using the same filter cloth as in Example 1 to separate a catalyst. The potassium concentration in the filtrate was 0.2% by mass. Moreover, simple distillation of the filtrate was performed under a reduced pressure of 0.06 kPaA to obtain 3.34 kg (13.1 mol) of 1,12-dodecanediol divinyl ether (hereinafter referred to as 3DVE). The obtained 3DVE had a purity of 99% or more and a yield of 67%. The results are shown in Table 1.

Example 4: Synthesis of 3-methyl-1,5-pentanediol divinyl ether (MPDVE)

Into the same reaction vessel as in Example 1, 3.40 kg (28.8 mol) of 3-methyl-1,5-pentanediol (manufactured by FUJIFILM Wako Chemical Corporation) and 0.34 kg (6.0 mol) of potassium hydroxide were charged, and nitrogen was purged inside the vessel.

After nitrogen purge, the temperature inside the vessel was raised to 110°C and stirring was performed at 250 rpm. The pressure inside the vessel was gradually reduced to 5.5 kPaA, and bubbling with 0.1 NL/min nitrogen was performed for 3 hr from the liquid sampling line. Through the above operation, 0.11 kg of a distillate including water as the main component was taken out. Thus, potassium alkoxide derived from 3-methyl-1,5-pentanediol was obtained.

Then, the temperature in the vessel was raised to 155°C, and the inside of the vessel was purged with acetylene while stirring at 420 rpm. Acetylene was continuously supplied, the inside of the vessel was kept at 0.03 MPaG and 155°C, and the reaction was performed for 10 hr. Then, the temperature inside the vessel was lowered to 135°C, and the reaction was further performed for 3.5 hr. As a result of gas chromatography analysis of the reaction solution, the conversion rate was 99% or more, and the selectivity was 91%. The reaction rate in this case was calculated to be 62 g/L·hr, and the production rate in reaction step was calculated to be 51 g/L·hr.

This reaction solution was subjected to filtration under pressure at 120°C and a nitrogen pressure of 0.1 MPaG by using the same filter cloth as in Example 1 to separate a catalyst. The potassium concentration in the filtrate was

0.2% by mass. Moreover, simple distillation of the filtrate was performed under a reduced pressure of 1.3 kPaA to obtain 3.41 kg (20.0 mol) of 3-methyl-1,5-pentanediol divinyl ether (hereinafter referred to as MPDVE). The obtained MPDVE had a purity of 99% or more and a yield of 70%. The results are shown in Table 1.

Comparative Example 1: Synthesis of 1,12-octadecanediol divinyl ether (ODDVE)

Into the same reaction vessel as in Example 1, 0.17 kg (3.0 mol) of potassium hydroxide, 1.50 kg (5.2 mol) of 1,12-octadecanediol that had previously dissolved by heating, and 3.50 kg of dimethyl sulfoxide (Nippon Refine Co., Ltd.) were charged, and nitrogen was purged inside the vessel.

While stirring the inside of the vessel at 420 rpm, the temperature was raised to 80°C, and then the inside of the vessel was purged with acetylene. Acetylene was continuously supplied, the pressure inside the vessel was kept at 0.03 MPaG, and the reaction was performed for 13.5 hr at a temperature inside the vessel of 80°C. As a result of gas chromatography analysis of the reaction solution, the conversion rate was 99% or more, and the selectivity was 89%. The reaction rate in this case was calculated to be 22 g/L·hr, and the production rate in reaction step was calculated to be 22 g/L·hr.

This reaction solution was transferred to a 10 L plastic container and allowed to stand for liquid separation, and then the upper layer was removed. The potassium concentration in the upper layer liquid was 0.5% by mass. Moreover, simple distillation of the upper layer liquid was performed under a reduced pressure of 0.02 kPaA to obtain 1.28 kg (3.8 mol) of ODDVE. The obtained ODDVE had a purity of 99% or more and a yield of 72%. The results are shown in Table 1.

Comparative Example 2: Synthesis of 2,4-diethyl-1,5-pentanediol divinyl ether (DEPDVE)

Into the same reaction vessel as in Example 1, 1.00 kg (6.3 mol) of 2,4-diethyl-1,5-pentanediol, 0.10 kg (1.8 mol) of potassium hydroxide, and 4.00 kg of dimethyl sulfoxide were charged, and nitrogen was purged inside the vessel.

While stirring the inside of the vessel at 420 rpm, the temperature was raised to 80°C, and then the inside of the vessel was purged with acetylene. Acetylene was continuously supplied, the pressure inside the vessel was kept at 0.03 MPaG, and the reaction was performed for 7 hr at a temperature of 80°C inside the vessel. As a result of gas chromatography analysis of the reaction solution, the conversion rate was 99% or more, and the selectivity was 90%. The reaction rate in this case was calculated to be 32 g/L·hr, and the production rate in reaction step was calculated to be 32 g/L·hr.

This reaction solution was transferred to a 20 L plastic container, 5.20 kg of hexane was added, and the mixture was shaken and allowed to stand. After standing, the upper layer was removed and concentrated under reduced pressure. Moreover, fine distillation of the concentrated solution was performed by using a distillation column manufactured by Kiriyama Glass Works Co. with 10 theoretical stages. 0.87 kg (4.1 mol) of DEPDVE was obtained under the conditions of a pressure of 1.3 kPaA and a reflux ratio of 10. The obtained DEPDVE had a purity of 99% or more and a yield of 65%. The results are shown in Table 1.

[Table 1]

| | Targeted divinyl ether compound | Reaction solvent | r1 (g/L·hr) | r2 (g/L·hr) | Catalyst separation | Distillation | Yield (%) |
|---|---|---|---|---|---|---|---|
| Example 1 | ODDVE | - | 61 | 49 | Filtration | Simple distillation | 81 |
| Example 2 | DEPDVE | - | 60 | 47 | Filtration | 5 stages | 70 |
| Example 3 | 3DVE | - | 52 | 44 | Filtration | Simple distillation | 67 |
| Example 4 | MPDVE | - | 62 | 51 | Filtration | Simple distillation | 70 |
| Comparative Example 1 | ODDVE | DMSO | 22 | 22 | Liquid separation | Simple distillation | 72 |
| Comparative Example 2 | DEPDVE | DMSO | 32 | 32 | Extraction | 10 stages | 65 |

The symbols in the table indicate the following.

r1: Reaction rate
r2: Production rate in reaction step
ODDVE: 1,12-Octadecanediol divinyl ether
DEPDVE: 2,4-Diethyl-1,5-pentanediol divinyl ether
3DVE: 1,12-Dodecanediol divinyl ether
MPDVE: 3-Methyl-1,5-pentanediol divinyl ether
DMSO: Dimethyl sulfoxide

As shown in Table 1, the reaction rate and the production rate in reaction step were significantly increased by reacting the above alkanediol with acetylene in the absence of a solvent. Moreover, the yield was confirmed to be high. In the Leppe method, it generally considers that the reaction rate is affected by the solubility of acetylene in the reaction system, and therefore the reaction rate in the presence of the solvent is higher than that of solvent-free condition because of higher acetylene solubility. To such general consideration, it is surprising that the solvent-free condition provides higher reaction rate as described above.

## Claims

1. A method for reacting a compound represented by formula (1) with acetylene by using an alkali metal catalyst to produce a compound represented by formula (2), wherein the reaction is performed in the absence of a solvent,

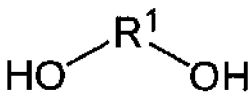
(1)

wherein $R^1$ represents an alkylene group having 4 to 20 carbon atoms, and

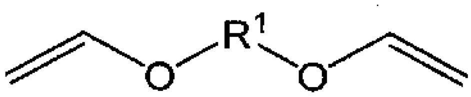
(2)

wherein $R^1$ has the same meaning as $R^1$ in formula (1).

2. The production method according to claim 1, wherein the compound represented by formula (1) is represented by formula (3), (4), or (5),

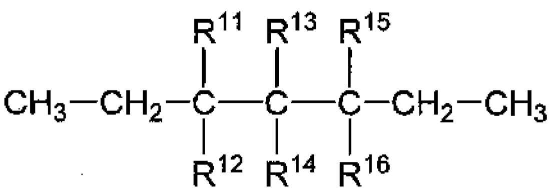
(3)

wherein any two of $R^{11}$ to $R^{16}$ represent a hydroxy group or a hydroxymethyl group and the others represent a hydrogen atom,

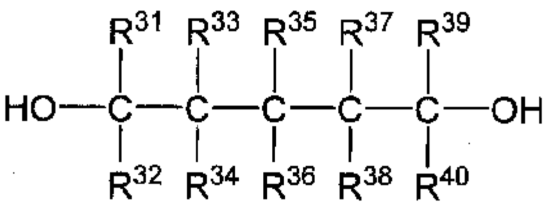
(4)

wherein any one of $R^{31}$ to $R^{40}$ represents a methyl group and the others represent a hydrogen atom, and

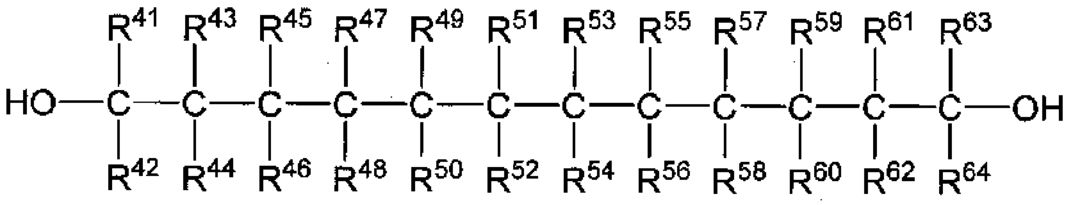

(5)

wherein $R^{41}$ to $R^{64}$ represent a hydrogen atom or an alkyl group having 1 to 8 carbon atoms, provided that as for a combination of $R^{41}$ to $R^{64}$, any one of $R^{41}$ to $R^{64}$ is an alkyl group having 1 to 8 carbon atoms and the others are hydrogen atoms, or all of $R^{41}$ to $R^{64}$ are hydrogen atoms.

**3.** The production method according to claim 1 or 2, wherein the compound represented by formula (1) is at least one selected from the group consisting of 3-methyl-1,5-pentanediol, 2,4-diethyl-1,5-pentanediol, 2-butyl-2-ethyl-1,3-propanediol, 1,12-dodecanediol, and 1,12-octadecanediol.

**4.** The production method according to any one of claims 1 to 3, wherein the reaction is performed in a range of 50 to 170°C.

**5.** The production method according to any one of claims 1 to 4, wherein the alkali metal catalyst is at least one selected from the group consisting of an alkali metal hydroxide and an alkali metal carbonate.

**6.** The production method according to any one of claims 1 to 5, wherein an amount of the alkali metal catalyst used is in a range of 1 to 60 mol with respect to 100 mol of the compound represented by formula (1).

**INTERNATIONAL SEARCH REPORT**

International application No. PCT/JP2019/046071

A. CLASSIFICATION OF SUBJECT MATTER
C07C 41/08(2006.01)i; C07C 43/16(2006.01)i; C07B 61/00(2006.01)n; B01J23/04(2006.01)i
FI: C07C41/08; C07C43/16; B01J23/04 Z; C07B61/00 300

According to International Patent Classification (IPC) or to both national classification and IPC

B. FIELDS SEARCHED

Minimum documentation searched (classification system followed by classification symbols)
C07C41/08; C07C43/16; C07B61/00; B01J23/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN), CASREACT (STN)

C. DOCUMENTS CONSIDERED TO BE RELEVANT

| Category* | Citation of document, with indication, where appropriate, of the relevant passages | Relevant to claim No. |
|---|---|---|
| X | CRIVELLO, James V.; MCGRATH, Thomas M., "Photoinitiated alternating copolymerization of dialkyl maleates and fumarates with vinyl ethers", Journal of Polymer Science, Part A: Polymer Chemistry, 07 September 2010, vol. 48, no. 21, pp. 4726-4736, page 4727, right column, General Preparation of Difunctional Vinyl Ethers | 1-6 |
| X | DE 19924159 A1 (BASF AG) 30.11.2000 (2000-11-30) examples 7, 9, claims | 1-6 |
| X | JP 5-279284 A (NIPPON CARBIDE INDUSTRIES CO., INC.) 26.10.1993 (1993-10-26) paragraphs [0017]-[0019], [0022]-[0029] | 1-6 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 February 2020 (19.02.2020) | 03 March 2020 (03.03.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/046071

C (Continuation). DOCUMENTS CONSIDERED TO BE RELEVANT

| Category* | Citation of document, with indication, where appropriate, of the relevant passages | Relevant to claim No. |
|---|---|---|
| X | 国近三吾、 榊原保正，1,4-ブタンジオール-モノビニルエーテルの合成とその重合，工業化学雑誌，1957, vol. 60, no. 6, pp. 761-763, page 761, right column to page 762, left column, vinylation of 1,4-butanediol, table 1, experiment number 4, (KUNICHIKA, Sango, SAKAKIBARA, Yasumasa, "Preparation and Polymerization of 1,4-Butanediol-Monovinyl Ether", The Journal of the Society of Chemical Industry, Japan) | 1-6 |
| X | DE 2628408 A1 (BASF AG) 12.01.1978 (1978-01-12) examples 1, 3, claims | 1-6 |
| X | JP 4-500520 A (EXFLUOR RESEARCH CORPORATION) 30.01.1902 (1992-01-30) example 39 | 1-6 |
| X | JP 8-225479 A (BASF AG.) 03.09.1996 (1996-09-03) claims, paragraphs [0014], [0021], [0030]-[0035] | 1-6 |

Form PCT/ISA/210 (continuation of second sheet) (January 2015)

**INTERNATIONAL SEARCH REPORT**
Information on patent family members

International application No.
PCT/JP2019/046071

| Patent Documents referred in the Report | Publication Date | Patent Family | Publication Date |
|---|---|---|---|
| DE 19924159 A1 | 30 Nov. 2000 | (Family: none) | |
| JP 5-279284 A | 26 Oct. 1993 | (Family: none) | |
| DE 2628408 A1 | 12 Jan. 1978 | (Family: none) | |
| JP 4-500520 A | 30 Jan. 1992 | US 5322904 A<br>example 39<br>WO 1990/003353 A1<br>EP 441807 A1<br>KR 10-1995-0034940 A | |
| JP 8-225479 A | 03 Sep. 1996 | US 5723685 A<br>claims, column 2 lines 15-19, 47-53, examples<br>EP 709359 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014513048 A
- WO 2015190376 A
- JP 2017068246 A